# EUROPEAN PATENT SPECIFICATION

(11) **EP 2 210 638 B1**
(45) Date of publication and mention of the grant of the patent: **27.03.2013**
(21) Application number: 09001001.8
(22) Date of filing: 26.01.2009
(51) Int. Cl.: A61M 15/00

(54) **Inhaler**
Inhalator
Inhalateur

(43) Date of publication of application: 28.07.2010
(73) Proprietor: Boehringer Ingelheim International GmbH, 55216 Ingelheim am Rhein (DE)
(72) Inventor: Besseler, Jens, 55216 Ingelheim am Rhein (DE); Jung, Andree, 55216 Ingelheim am Rhein (DE); Krakowka, Manuel, 55216 Ingelheim am Rhein (DE); Wachtel, Herbert, 55216 Ingelheim am Rhein (DE)
(74) Representative: Von Rohr

(56) References cited:
- WO-A-01/26720
- WO-A-2006/108877
- GB-A- 2 405 798
- GB-A- 2 407 042
- US-A- 6 089 228

## Description

The present invention relates to an inhaler according to the preamble of claim 1.

The present invention relates to an inhaler for delivery of a powder-form inhalation formulation from a blister strip with a plurality of blister pockets (also called blisters) containing the inhalation formulation in doses.

GB 2 407 042 A discloses an inhaler with a rolled-up blister strip. The inhaler comprises a manually operated, pivotable actuator, which operates a conveyor for stepwise moving the blister strip. The actuator supports a piercer and an associated mouthpiece. By pivoting the actuator, the blister strip and be moved forward and blister pockets of the blister strip can be pierced one after the other. When a patient breathes in an air stream passes through the previously pierced blister pocket, with the result that the inhalation formulation in the blister pocket mixes with the air and is discharged to the patient.

WO 2006/108877 A2 discloses a blister piercing element for a dry powder inhaler. The element includes a pair of piercing heads, wherein the heads are in a back to back position. Each piercing head is in the form of a blade like element comprising a first leg portion which extends in the direction of insertion, or which is only angled away from the direction of insertion by a small extend. A second leg extends from the end of the first leg in a more lateral direction.

The present invention relates to passive inhalers, i.e. inhalers where the patient or user breathes in to generate an air stream, which entrains the inhalation formulation and forms the desired aerosol.

The design of a piercing member for puncturing the blister pockets is important in order to achieve good discharge characteristics. Multiple piercing member designs are known. However, studies have shown that the present piercer designs do not result in optimized discharge characteristics and/or are difficult to produce and/or have a complicated design.

Object of the present invention is to provide an inhaler with optimized discharge characteristics and/or simple design.

The above object is achieved by an inhaler according to claim 1. Advantageous embodiments are subject of the subclaims.

In the present invention, the piercing member comprises two piercing elements extending in V-shape away from each other. The V-shape of the piercing elements result in that two main flaps of the lid or any other cover are formed when a receptacle is pierced. In particular, the flaps are bent by the piercing elements towards each other and/or away from opposite ends of the pierced receptacle.

According to one aspect of the present invention, the piercing member comprises only these two piercing elements for forming only two separate openings in the lid of a receptacle, such as a blister pocket, that is to be opened. In this case, the piercing elements are at least essentially flat, preferable plate-like. This allows optimized discharge characteristics and a simple design.

According to another aspect of the present invention, the piercing elements arranged in V-shape are spaced from each other. In particular, a contact member is arranged between the piercing elements so that it can contact the lid in an area between separate openings formed by the piercing elements in the lid. This allows optimized discharge characteristics and a simple design.

Preferably, the contact member is interconnected with the piercing elements, in particular supports or holds the piercing elements, preferably exclusively.
Preferably, the contact member is longitudinal and/or ridge- or bridge-like.

It is especially preferred to form the piercing member and/or piercing elements from sheet material, in particular by cutting the sheet material so that the piercing elements can be bent into the desired V-shape, wherein the piercing elements are interconnected by a common member, in particular the contact member.

In particular, the lid is only punctured by the piercing member. Preferably, the piercing member comprises only these two piercing members.

The different aspects and features of the present invention can be realized independently from each other and in any combination.

Further aspects, features, properties and advantages of the present invention are described in the claims and the subsequent description of a preferred embodiment, with reference to the drawing. There are shown in:
- Fig. 1: a schematic sectional view of an inhaler without mouthpiece cover;
- Fig. 2: a schematic sectional representation of the inhaler with closed mouthpiece cover;
- Fig. 3: an enlarged sectional view of the inhaler in the region of a mouthpiece and a piercing member; and
- Fig. 4: a perspective view of the piercing member with an insert seen from the piercing side.

In the Figures, same reference numbers are used for identical or similar parts, even if a repeated description is omitted. In particular identical or corresponding advantages and properties then also result or may be achieved.

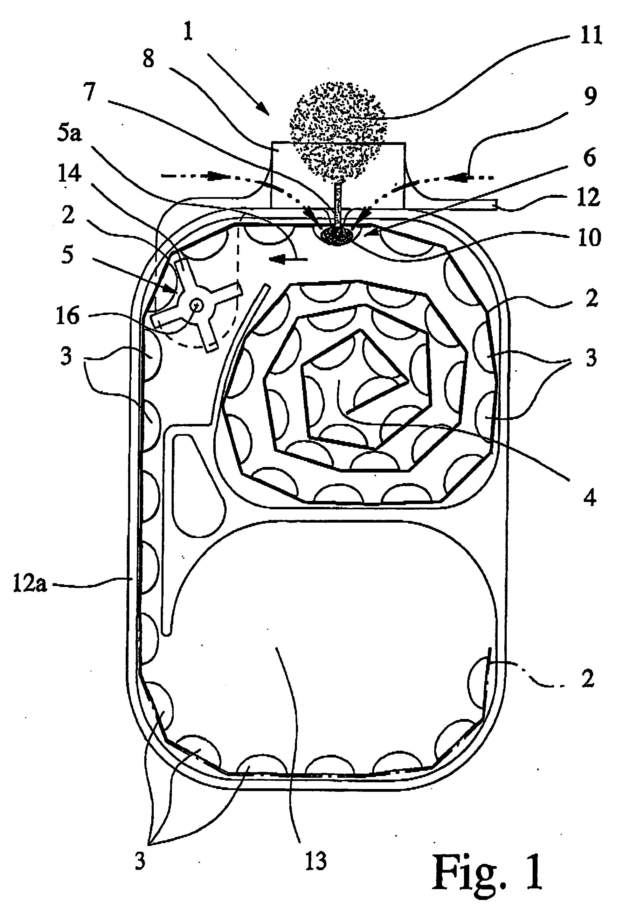

Fig. 1 shows in a very schematic sectional representation an inhaler 1.

The inhaler 1 serves to deliver a powdered inhalation formulation from a preferably band-shaped carrier, such as a blister strip 2. The blister strip 2 is finite, not forming an endless or closed loop. It has a large number of blister pockets 3 respectively containing directly a dose of the loose inhalation formulation, in particular powder 10. Thus, the formulation is pre-metered.

The inhaler 1 has a reservoir 4 for the still unused blister strip 2 with closed (sealed) receptacles, here blister pockets 3. The blister strip 2 is preferably rolled up or wound up in the reservoir 4. In the representation example the reservoir 4 is formed such that the blister strip 2 can be moved outwards or pulled out of the reservoir 4 as easily as possible.

In the representation example the blister strip 2 is directly received in the reservoir 4. However, instead of this a cassette, a container, a drum or suchlike can also be fitted or inserted with the blister strip 2 into the inhaler 1 or the reservoir 4.

The inhaler 1 has preferably a conveyor 5 for stepwise onward movement of the blister strip 2 in direction of arrow 5a by one blister pocket 3, in order to feed the blister pockets 3 successively to an opening and/or removal position 6 where the respective blister pocket 3 is opened and can be emptied.

The blister pockets 3 can be opened respectively preferably by means of a piercing member 7, which punctures or cuts open a lid of the respectively aligned blister pocket 3 in position 6. The piercing member 7 is hollow and/or in fluid connection with an adjacent mouthpiece 8 of the inhaler 1.

During or for inhalation a patient or user, not represented, places the mouthpiece 8 in his mouth and breathes in. The respectively opened blister pocket 3, into which the piercing member 7 partly extends, is thereby emptied by sucking in. An air stream 9 of ambient air is sucked in and passed through the opened blister pocket 3 such that the loose powder 10 (forming the inhalation formulation and being schematically shown in Fig. 1 only in the actually opened blister pocket 3 below mouthpiece 8) is dispensed with the sucked-in ambient air as an aerosol cloud 11 via the mouthpiece 8. This situation is schematically represented in Fig. 1.

The inhaler I has a preferably manually actuatable, lever-like actuator 12 being pivotally mounted to a housing 12a of the inhaler 1. The piercing member 7 and the mouthpiece 8 are attached to and supported by the actuator 12.

The actuator 12 is operable (pivotable) to cause the piercing member 7 to puncture the lid of the respectively aligned blister pocket 3 in position 6 below the mouthpiece 8.

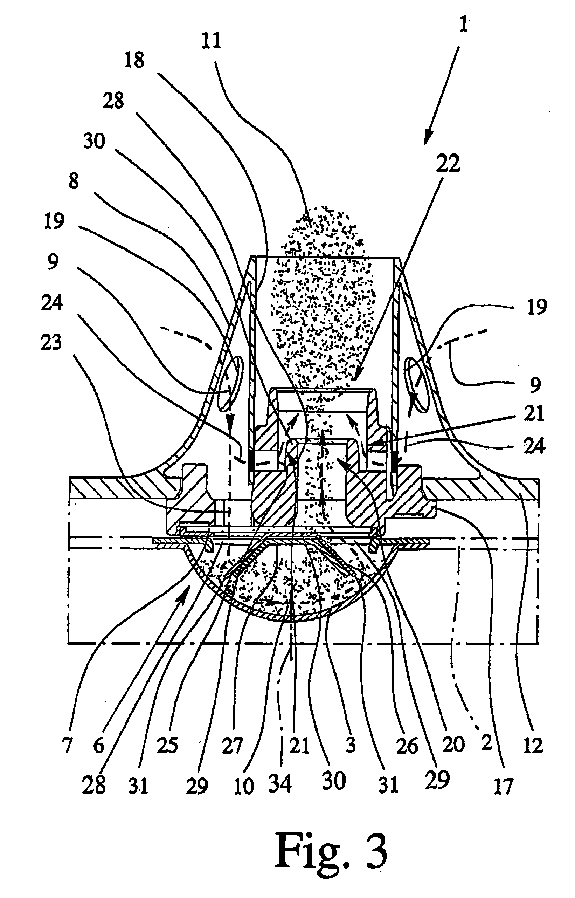

When the actuator 12 swivels from the position shown in Fig. 1 (here anticlockwise) to the partially opened position shown in Fig. 3, the piercing member 7 is withdrawn from the last-pierced blister pocket 3.

Then, the blister strip 2 is moved forward by one blister pocket 3, so that the next blister pocket 3 is moved in position 6. This will be explained in more detail later.

When the actuator 12 swivels back into the position shown in Fig. 1, i.e. is manually moved back, the next aligned blister pocket 3 of the blister strip 2 is punctured by the piercing member 7 and thereby opened. Then, the next inhalation can take place, i.e. the inhaler 1 is activated.

The inhaler 1 has preferably a receiving space or apparatus 13 to receive or store the used part of the blister strip 2. The receiving space or apparatus 13 is formed such that the used part can be wound up. Fig. 1 shows a situation with essentially filled reservoir 4 and still essentially empty receiving space 13.

The conveyor 5 comprises preferably a conveying wheel 14, which can engage between the blister pockets 3 and thus convey the blister strip 2 in form-locking or form-fit manner. This allows very secure or precise moving or indexing of the blister strip 2 as desired and/or necessary.

The conveyor 5 or its conveying wheel 14 is arranged preferably between the reservoir 4 and the receiving apparatus 13, in particular between the removal position 6 and the receiving apparatus 13, thus after the emptying of the blister pockets 3.

The pivot axis of the actuator or lever 12 is coaxial with the rotation axis of the conveying wheel 14. In particular, the actuator or lever 12 may be supported by an axle of the conveying wheel 14.

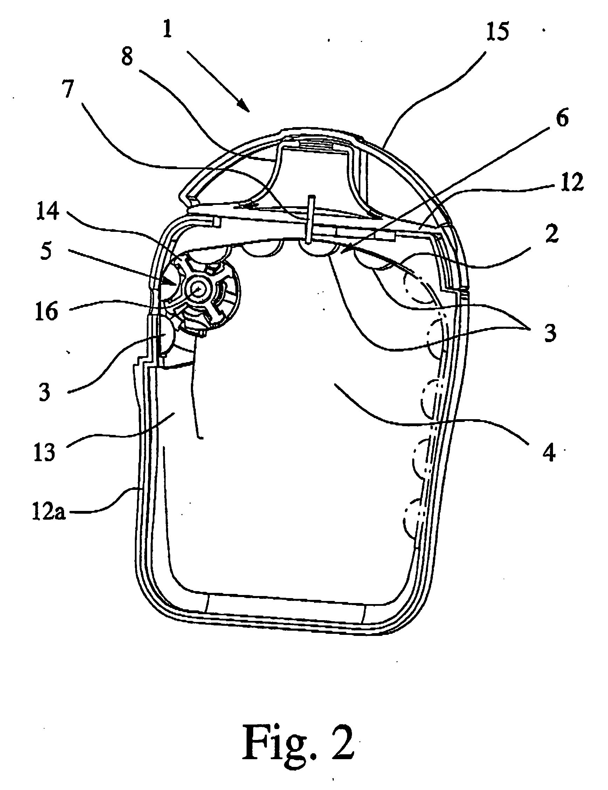

The inhaler 1 comprises a mouthpiece cover 15. The mouthpiece cover 15 is not shown in Fig. 1, which explains only the basic principle of the inhaler 1, but in Fig. 2, which shows a more realistic, but still very schematic sectional view of the inhaler 1. Fig. 2 shows the inhaler 1 with closed mouthpiece cover 15, wherein the blister strip 2 has been partly omitted for illustration purposes.

The mouthpiece cover 15 is pivotable around a cover axis 16, which is indicated in Fig. 2 and 3 and extends perpendicular to the drawing plane in the present representation.

The pivot axis of the actuator 12 extends preferably coaxial to or with the cover axis 16. The rotation axis of the conveying wheel 14 extends preferably coaxial to the cover axis 16 and to the pivot axis of the actuator 12.

The conveyor 5 or its conveying wheel 14 is preferably driven by the mouthpiece cover 15, namely by the pivotal movement of the mouthpiece cover 15. In particular, the blister strip 2 is moved forward, when the mouthpiece cover 15 is opened. Preferably, only part of the opening movement of the mouthpiece cover 15 actuates or operates the conveyor 5 or its conveying wheel 14 to move the blister strip 2 forward.

When the mouthpiece cover 15 is opened starting with the completely closed position shown in Fig. 2, in a first phase of the opening movement, for example up to a first angle of about 10, 20 or 30 degrees, the blister strip 2 is not moved due to a respective freewheel (not shown) between the mouthpiece cover 15 and the conveying wheel 14.

First of all, the actuator 12 has to be moved or opened in order to withdraw the piercing member 7 from the previously pierced and usually already emptied blister pocket 3. This opening movement of the actuator 12 can be performed manually. However, the actuator 12 preferably opens automatically when opening the mouthpiece cover 15. In particular, the mouthpiece cover 15 can be opened up to the first angle. When the mouthpiece cover 15 reaches this angle, e.g. about 20 degrees, the actuator 12 flips automatically open into its opened position, in particular due to a biasing or spring means (not shown) or the like. However, it also possible that the actuator 12 moves jointly with the mouthpiece cover 15 in the first phase of the opening movement (e. g. due to a ratchet mechanism, spring or the like) until the actuator 12 reaches its opened position or the first angle.

The opened position of the actuator 12 is preferably set such that the piercing member 7 is not exposed to the exterior and/or that the inhaler 1 is not completely opened in order to avoid or at least minimize any potential external influences.

In order to limit the open position of the actuator 12, the opening or pivot range of the actuator 12 is smaller than the one of the mouthpiece cover 15 and/or is restricted to preferably at most 20 degrees, in particular to about 10 degrees or less.

However, it is also possible that the actuator 12 is not limited in its opening position, but can open or pivot as far as the mouthpiece cover 15, in particular jointly with the mouthpiece cover 15.

During the further opening (second phase) of the mouthpiece cover 15, the conveyor 5 or its conveying wheel 14 is actuated to move or index the blister strip 2 by one blister pocket 3 onward to the next blister pocket 3 that shall be emptied. This blister movement happens preferably up to the complete opening of the mouthpiece cover 15.

Only when the mouthpiece cover 15 is opened completely, i.e. reaches its end position, the movement of the blister strip 2 may be set or fixed by a respective mechanism (not shown) and/or decoupled from the mouthpiece cover movement to keep the next blister pocket 3 in position 6 for puncturing. However, if the mouthpiece cover 15 is not fully opened and closed again, then, the blister strip 2 is moved backward. This facilitates operation of the inhaler 1 and, in particular, prohibits that incomplete or unintended operation of the mouthpiece cover 15 results in an undesired movement of the blister strip 2 and eventually in an undesired opening of the next blister pocket 3.

Preferably, a lock (not shown) is provided so that the opened actuator 12 can be closed again only if the mouthpiece cover 15 has been fully opened or pivoted back to the first angle or the last-pierced blister pocket 3 has been moved back into position 6. Thus, the piercing member 7 cannot be pushed against an area of the blister strip 2 without or beside a blister pocket 3.

When the mouthpiece cover 15 has been fully opened and the next blister pocket 3 has been moved in position 6, the actuator 12 can be pivoted back, i.e. closed, in order to pierce the already aligned, still closed blister pocket 3. Then, the inhaler 1 is ready for inhalation, i.e. activated as already described.

After inhalation, the inhaler 1 can be closed by pivoting back the mouthpiece cover 15 into its closed position.

In order to operate the conveyor 5 or its conveying wheel 14 by movement of the mouthpiece cover 15 as described above or in any other suitable manner, the mouthpiece cover 15 is coupled with the conveyor 5, in particular the conveying wheel 14, via the already mentioned freewheel, a suitable transmission, a slipping clutch and/or any other suitable coupling or the like. Preferably, the freewheel, transmission, coupling or the like is integrated into or located adjacent to the conveying wheel 14 or a respective axle.

Preferably, the mouthpiece cover 15 covers axially an axle or the axis of the actuator and/or conveying wheel 14.

Fig. 3 shows in an enlarged sectional view the piercing member 7 with the mouthpiece and an opened blister pocket 3 in the removal position 6. It can be seen that the piercing member 7 or inhaler 1 preferably comprises an insert 17, which is connected to the mouthpiece 8 and, in particular, extends into an outlet space or tube 18 of the mouthpiece 8.

Preferably, the insert 17 is held form-fit within the mouthpiece 8 or its outlet tube 18. However, other construction solutions are also possible.

The insert 17 is located adjacent to the piercing member 7. In particular, the piercing member 7 forms or holds the insert 17 or vice versa.

The piercing member 7 is connected preferably with the actuator 12, in particular via the insert 17 and/or the mouthpiece 8 as shown in Fig. 3.

The inhaler 1 or mouthpiece 8 comprises preferably at least one, here multiple air openings 19 through which the air stream 9 of ambient air can flow in.

The inhaler 1, the piercing member 7, the mouthpiece 8 and/or the insert 17 form a feeding path 20 for the air which has been flown through the opened blister pocket 3 and, in addition, preferably a bypass path 21 for air bypassing the blister pocket 3. Both paths 20 and 21 end preferably within the mouthpiece 8 or its outlet tube 18 and/or at a mixing zone 22 where the respective streams through the paths 20 and 21 mix.

In particular, the air stream 9 entering through the air openings 19 is split up into a feeding air stream 23 flowing through the opened blister pocket 3 and then through the feeding path 20, and into a bypass air stream 24 flowing through the bypass path 21.

Fig. 3 shows schematically the aerosol generation when the air flows. The feeding air stream 23 flowing through the opened blister pocket 3 entrains the inhalation formulation (powder 10) and flows into the mouthpiece 8 or its outlet tube 18, in particular to the mixing zone 22 where it mixes with the bypass air stream 24. Thus, the aerosol cloud 19 is generated as schematically shown in Fig. 3.

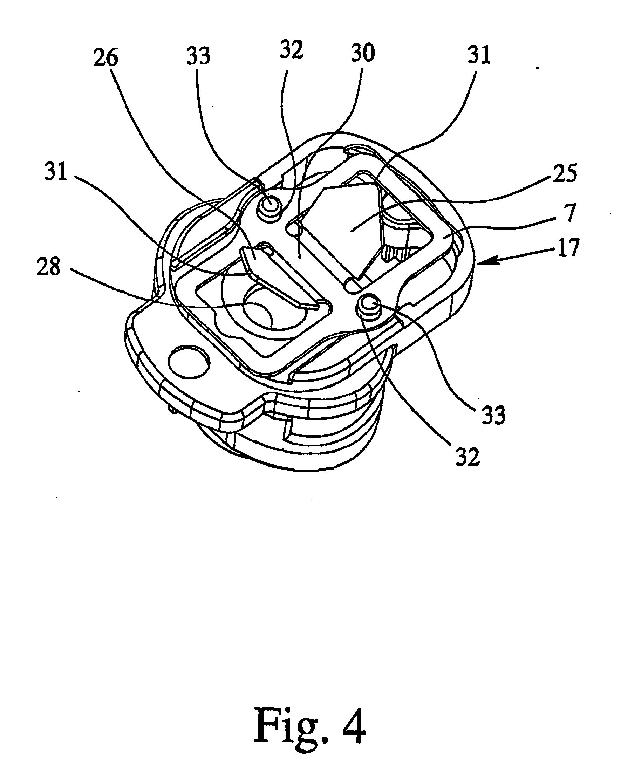

In the present embodiment, the piercing member 7 comprises two piercing elements 25 and 26 as shown in Fig. 3 and in the perspective view of the piercing member 7 according to Fig. 4.

The first piercing element 25 serves to form a first opening 28 (inlet opening) in the lid 27 of the blister pocket 3 as shown in Fig. 3. The second piercing element 26 forms a separate, second opening 29 (outlet opening) in the lid 27 as schematically shown in Fig. 3. Thus, the feeding air stream 23 can flow in through the first opening 28 and out through the second opening 29. The second opening 29 is fluidically connected to the feeding path 20, which is formed here preferably by a channel within the piercing member 7 and/or insert 17. This channel opens to the mixing zone 22.

In the present embodiment, the bypass path 21 leads through or is formed by a preferably annular bypass channel, preferably formed by the insert 17, to the mixing zone 22. In particular, the bypass channel surround concentrically or radially the channel of feeding path 20. However, other constructional solutions are possible.

In particular, the piercing member 7 is unitary and/or essentially plate-like and/or comprises or forms the two piercing elements 25, 26 protruding and extending towards the blister pocket 3 to be opened.

Preferably, the piercing member 7 comprises only two piercing elements 25, 26 for puncturing the lid 27, i.e. for opening a blister pocket 3 in position 6, in particular for forming the two separate openings 28, 29 in the lid 27.

Preferably, the piercing elements 25, 26 are at least essentially flat or plate-like.

According to the invention, the piercing elements 25, 26 run or extend at least essentially in V-shape away from each other. In particular, the piercing elements 25, 26 are inclined away from each other, so that the distance between then increases with increasing distance away from a plate forming the piercing member 7 or the lid 27 (when the piercing elements 25, 26 extend in to the blister pocket 3).

The piercing elements 25, 26 are inclined to a piercing plane 34 extending essentially in the direction of dispensing and/or essentially perpendicular to the lid 27 or any other cover of the receptacle / blister pocket 3.

Preferably, the main extension planes of the piercing elements 25, 26 and the piercing plane 34 comprise a common line (cross line) where all the planes cross each other. With other words, one piercing element could be rotated geometrically into the other piercing element or vice versa around this cross line. However, other orientations of the piercing elements 25, 26 are also possible.

The angle of the piercing elements 25, 26 to the piercing plane 34 is about 30 to 60, preferably about 40 to 50 degrees, i.e. that the angle between the piercing elements 25, 26 is preferably about 80 to 100 degrees.

Preferably, the piercing elements 25, 26 are inclined to the surface or main plane of the lid 27 or blister strip 2, to the longitudinal extension of the blister strip 2, to the onward movement 5a of the blister strip 2, and/or to the relative movement of the piercing member 7 to the blister pocket 3 during puncturing. In particular, the inclination is 30 to 60°, preferably about 40 to 50°, most preferably about 45°.

Preferably, the piercing elements 25, 26 are spaced from each other.

Preferably, a contact member 30 is arranged between the piercing elements 25, 26 so that the contact member 30 can contact the lid 27 in an unruptured area between the openings 28, 29 formed by the piercing elements 25, 26 in the lid 27.

Preferably, the contact member 30 comprises a flat or ridge-like contact area for directly contacting the lid 27 in the area between the openings 28, 29.

Preferably, the contact member 30 or its contact area extends at least over the entire width of the receptacle / blister pocket 3 or lid 27 to ensure a defined flow of the feeding air stream 23 through the first opening 28 into the blister pocket 3 and through the second opening 29 out of the blister pocket 3 and, in particular, to prevent any undesired leakage of feeding air between the lid 27 and the piercing member 7, i.e. to prevent that (a relevant part of) the feeding air stream 23 can bypass the opened receptacle / blister pocket 3.

Preferably, the piercing elements 25, 26 are interconnected or held or supported by a common member, in particular the contact member 30.

Preferably, the piercing elements 25, 26 are respectively tapered to its respective free ends and/or respectively comprise or form a tip 31.

Preferably, the piercing member 7 and/or piercing elements 25, 26 are made of sheet material, in particular metal sheet.

In the present embodiment, the piercing elements 25, 26 are bent into the respective inclined position.

Preferably, the piercing elements 25, 26 are only respectively connected to a flat, plate-like region of the piercing member 7 along an essentially linear edge.

Preferably, the piercing member 7 is made of metal and/or of sheet material. The piercing member 7 is preferably separately formed or made from the insert 17.

Preferably, the piercing member 7 is held in a form-fit manner by the insert 17 or any other suitable component of the inhaler 1. Preferably, the form-fit connection is achieved in that the piercing member 7 comprises at least one though hole 32 through which a protrusion 33 extends that is deformed at least at its free end to fix the piercing member 7. The protrusion 33 protrudes from the insert 17 or any other suitable component of the inhaler 1. The protrusion 33 is preferably made of plastic, so that the desired form-fit connection can be achieved in a simple manner, in particular by deforming, preferably due to pressure and/or heat. The through hole 32 is preferably located in a flat or plate-like region of the piercing member 7.

In the present embodiment, the piercing member 7 is preferably held or connected at at least two points, preferably by two protrusions 33 extending through two corresponding through holes 32.

It has to be noted that Fig. 3 shows only very schematically, in particular not in scale, a potential construction. Other constructional solutions are possible as a wall.

Preferably, the inhaler 1 is portable, works only mechanically and/or is handheld.

Preferably, the terms "blister strip" and "blister pockets" have to be understood in a very broad sense to cover also other kinds of storage means with receptacles or even bulk storages for the formulation.

### List of reference numbers

- 1: inhaler
- 2: blister strip
- 3: blister pocket
- 4: reservoir
- 5: conveyor
- 5a: onward movement
- 6: opening and/or removal position
- 7: piercing member
- 8: mouthpiece
- 9: air stream
- 10: powder
- 11: aerosol cloud
- 12: actuator
- 12a: housing
- 13: receiving apparatus
- 14: conveying wheel
- 15: mouthpiece cover
- 16: cover axis

- 17: insert
- 18: outlet tube
- 19: air opening
- 20: feeding path
- 21: bypass path
- 22: mixing zone
- 23: feeding air stream
- 24: bypass air stream
- 25: first piercing element
- 26: second piercing element
- 27: lid
- 28: first opening
- 29: second opening
- 30: contact member
- 31: tip
- 32: through hole
- 33: protrusion
- 34: piercing plane

## Claims

1. Inhaler (1) for delivery of an inhalation formulation from a preferably band-shaped carrier, such as a blister strip (2), with a plurality of receptacles, such as blister pockets (3), containing the inhalation formulation in doses, comprising:
preferably a conveyor (5) for stepwise onward movement of the blister strip (2), and
a piercing member (7) to puncture a lid (27) of a receptacle to be opened,
the inhaler (1) being designed such that - preferably by breathing in during inhalation - an air stream (9) of ambient air can be sucked in or delivered in order to discharge the respective dose from an opened receptacle, wherein the piercing member (7) comprises two piercing elements (25, 26),
**characterized in**
**that** the piercing elements (25, 26) extend in V-shape away from each other and that the piercing member (7) is made of sheet material, wherein the piercing elements (25, 26) are formed by bending part of the sheet material into an inclined position.

2. Inhaler according to claim 1, **characterized in that** a contact member (30) being arranged between the piercing elements (25, 26) so that the contact member (30) contacts the lid (27) in an area between separate openings (28, 29) formed by the piercing elements (25, 26) in the lid (27).

3. Inhaler according to claim 1 or 2, **characterized in that** the piercing elements (25, 26) are inclined to: the lid (27) to be punctured, to the surface or main plane of the lid (27) or carrier, to the longitudinal extension of the carrier, to the onward movement (5a) of the carrier, and/or to the piercing movement of the piercing member (7) relative to the receptacle to be punctured.

4. Inhaler according to one of the preceding claims, **characterized in that** the piercing elements (25, 26) are tapered at its free ends.

5. Inhaler according to one of the preceding claims, **characterized in that** the piercing elements (25, 26) are inclined to each other by an angle of about 80 to 100 degrees.

6. Inhaler according to one of the preceding claims, **characterized in that** the sheet material is metal sheet, in particular spring steel or stainless steel.

7. Inhaler according to one of the preceding claims, **characterized in that** the piercing elements (25, 26) are spaced from each other.

8. Inhaler according to one of the preceding claims, **characterized in that** the contact member (30) comprises or forms a flat or ridge-like contact area for contacting the lid (27) in an unruptured area.

9. Inhaler according to one of the preceding claims, **characterized in that** the contact member (30) extends over the entire width of the carrier or receptacle that is open or to be opened.

10. Inhaler according to one of the preceding claims, **characterized in that** the piercing elements (25, 26) are held by or connected to a common, preferably flat member, in particular by the contact member (30).

11. Inhaler according to one of the preceding claims, **characterized in that** the piercing member (7) is essentially flat or plate-like and/or extends essentially perpendicular to a piercing movement of the piercing member (7) relative to the receptacle to be punctured.

12. Inhaler according to one of the preceding claims, **characterized in that** the inhaler (1) comprises a preferably molded and/or unitary insert (17) holding or forming the piercing member (7).

## Patentansprüche

1. Inhalator (1) zur Abgabe einer Inhalationsformulierung von einem vorzugsweise bandförmigen Träger, wie einem Blisterstreifen (2), mit mehreren Aufnahmen, wie Blistertaschen (3), die die Inhalationsformulierung in Dosen enthalten, aufweisend:
vorzugsweise eine Transporteinrichtung (5) für schrittweisen Weitertransport des Blisterstreifens (2), und
ein Anstechteil (7) zum Durchstechen eines Deckels (27) einer zu öffnenden Aufnahme,
wobei der Inhalator (1) so ausgeführt ist, dass - vorzugsweise durch Einatmen während der Inhalation - ein Luftstrom (9) aus Umgebungsluft angesaugt oder abgegeben werden kann, um die jeweilige Dosis aus einer geöffneten Aufnahme abzuführen, wobei das Anstechteil (7) zwei Anstechelemente (25, 26) aufweist,
**dadurch gekennzeichnet,**
**dass** sich die Anstechelemente (25, 26) V-förmig voneinander weg erstrecken und dass das Anstechteil (7) aus Plattenmaterial hergestellt ist, wobei die Anstechelemente (25, 26) durch Biegen eines Teils des Plattenmaterials in eine geneigte Position gebildet werden.

2. Inhalator nach Anspruch 1, **dadurch gekennzeichnet, dass** ein Kontaktteil (30) so zwischen den Anstechelementen (25, 26) angeordnet ist, dass das Kontaktteil (30) den Deckel (27) in einem Bereich zwischen getrennten Öffnungen (28, 29) berührt, die durch die Anstechelemente (25, 26) im Deckel gebildet werden.

3. Inhalator nach Anspruch 1 oder 2, **dadurch gekennzeichnet, dass** die Anstechelemente (25, 26) zu dem zu durchstechenden Deckel (27), zu der Fläche oder Hauptebene des Deckels (27) oder Trägers, zu der Längserstreckung des Trägers, zu der Weiterbewegung (5a) des Trägers und/oder zu der Anstechbewegung des Anstechteils (7) bezüglich der anzustechenden Aufnahme geneigt sind.

4. Inhalator nach einem der vorhergehenden Ansprüche, **dadurch gekennzeichnet, dass** die Anstechelemente (25, 26) sich an ihren freien Enden verjüngen.

5. Inhalator nach einem der vorhergehenden Ansprüche, **dadurch gekennzeichnet, dass** die Anstechelemente (25, 26) in einem Winkel von ca. 80 bis 100 Grad zueinander geneigt sind.

6. Inhalator nach einem der vorhergehenden Ansprüche, **dadurch gekennzeichnet, dass** es sich bei dem Plattenmaterial um Blech handelt, insbesondere Federstahl oder rostfreien Stahl.

7. Inhalator nach einem der vorhergehenden Ansprüche, **dadurch gekennzeichnet, dass** die Anstechelemente (25, 26) voneinander beabstandet sind.

8. Inhalator nach einem der vorhergehenden Ansprüche, **dadurch gekennzeichnet, dass** das Kontaktteil (30) einen flachen oder stegförmigen Kontaktbereich zur Berührung des Deckels (27) in einem nicht durchbrochenen Bereich aufweist oder bildet.

9. Inhalator nach einem der vorhergehenden Ansprüche, **dadurch gekennzeichnet, dass** sich das Kontaktteil (30) über die gesamte Breite des Trägers oder der Aufnahme, der/die offen ist oder geöffnet werden soll, erstreckt.

10. Inhalator nach einem der vorhergehenden Ansprüche, **dadurch gekennzeichnet, dass** die Anstechelemente (25, 26) durch ein gemeinsames, vorzugsweise flaches Teil, insbesondere durch das Kontaktteil (30), gehalten werden oder damit verbunden sind.

11. Inhalator nach einem der vorhergehenden Ansprüche, **dadurch gekennzeichnet, dass** das Anstechteil (7) im Wesentlichen flach oder plattenförmig ist und/oder sich im Wesentlichen senkrecht zu einer Anstechbewegung des Anstechteils (7) bezüglich der zu durchstechenden Aufnahme erstreckt.

12. Inhalator nach einem der vorhergehenden Ansprüche, **dadurch gekennzeichnet, dass** der Inhalator (1) einen vorzugsweise geformten, gegossen und/oder einteiligen Einsatz (17) aufweist, der das Anstechteil (7) hält oder bildet.

## Revendications

1. Inhalateur (1) destiné à distribuer une formulation pour inhalation à partir d'un support de préférence en forme de bande, tel qu'un ruban thermoformé (2), comprenant une pluralité de réceptacles tels que des alvéoles thermoformées (3), contenant la formulation pour inhalation en doses, comprenant :
de préférence un élément d'avance (5) pour assurer le mouvement d'avance pas à pas du ruban thermoformé (2), et
un organe de perçage (7) destiné à percer un couvercle (27) d'un réceptacle à ouvrir,
l'inhalateur (1) étant conçu de telle sorte que - de préférence en inspirant au cours de l'inhalation - un flux d'air (9) d'air ambiant puisse être aspiré ou distribué afin de décharger la dose respective depuis un réceptacle ouvert, l'organe de perçage (7) comprenant deux éléments de perçage (25, 26),
**caractérisé en ce que**
les éléments de perçage (25, 26) s'étendent en forme de V à l'écart l'un de l'autre et **en ce que** l'organe de perçage (7) est fabriqué en un matériau en feuille, les éléments de perçage (25, 26) étant formés en courbant une partie du matériau en feuille dans une position inclinée.

2. Inhalateur selon la revendication 1, **caractérisé en ce qu'**un organe de contact (30) est agencé entre les éléments de perçage (25, 26) de telle sorte que l'organe de contact (30) vienne en contact avec le couvercle (27) dans une zone entre des ouvertures séparées (28, 29) formées par les éléments de perçage (25, 26) dans le couvercle (27).

3. Inhalateur selon la revendication 1 ou 2, **caractérisé en ce que** les éléments de perçage (25, 26) sont inclinés vers : le couvercle (27) à percer, vers la surface ou le plan principal du couvercle (27) ou du support, vers l'extension longitudinale du support, vers le mouvement d'avance (5a) du support, et/ou vers le mouvement de perçage de l'organe de perçage (7) par rapport au réceptacle à percer.

4. Inhalateur selon l'une quelconque des revendications précédentes, **caractérisé en ce que** les éléments de perçage (25, 26) sont effilés à leurs extrémités libres.

5. Inhalateur selon l'une quelconque des revendications précédentes, **caractérisé en ce que** les éléments de perçage (25, 26) sont inclinés l'un vers l'autre d'un angle d'environ 80 à 100 degrés.

6. Inhalateur selon l'une quelconque des revendications précédentes, **caractérisé en ce que** le matériau en feuille est une tôle métallique, en particulier en acier à ressort ou en acier inoxydable,

7. Inhalateur selon l'une quelconque des revendications précédentes, **caractérisé en ce que** les éléments de perçage (25, 26) sont espacés l'un de l'autre.

8. Inhalateur selon l'une quelconque des revendications précédentes, **caractérisé en ce que** l'organe de contact (30) comprend ou forme une zone de contact plate ou en forme de crête pour venir en contact avec le couvercle (27) dans une zone non rompue.

9. Inhalateur selon l'une quelconque des revendications précédentes, **caractérisé en ce que** l'organe de contact (30) s'étend sur toute la largeur du support ou réceptacle qui est ouvert ou qui doit être ouvert.

10. Inhalateur selon l'une quelconque des revendications précédentes, **caractérisé en ce que** les éléments de perçage (25, 26) sont retenus par ou connectés à un organe commun, de préférence plat, en particulier par l'organe de contact (30).

11. Inhalateur selon l'une quelconque des revendications précédentes, **caractérisé en ce que** l'organe de perçage (7) est essentiellement plat ou en forme de plateau et/ou s'étend essentiellement perpendiculairement à un mouvement de perçage de l'organe de perçage (7) par rapport au réceptacle à percer.

12. Inhalateur selon l'une quelconque des revendications précédentes, **caractérisé en ce que** l'inhalateur (1) comprend un insert de préférence moulé et/ou unitaire (17) retenant ou formant l'organe de perçage (7).
